# EUROPÄISCHE PATENTSCHRIFT

(11) **EP 0 433 485 B1**
(45) Veröffentlichungstag und Bekanntmachung des Hinweises auf die Patenterteilung: **19.05.1993**
(21) Anmeldenummer: 89123701.8
(22) Anmeldetag: 21.12.1989
(51) Int. Cl.: A61M 5/142

(54) **Anordnung zum transkutanen Ein- oder Nachfüllen von flüssigen Medikamenten in ein implantierbares Medikamentendosiergerät**
Device for filling or refilling of a liquid drug in an implantable drug dosage apparatus
Dispositif pour remplir ou recharger de médicaments liquides un dispositif de dosage de médicaments implantable

(43) Veröffentlichungstag der Anmeldung: 26.06.1991
(73) Patentinhaber: SIEMENS AKTIENGESELLSCHAFT, 80333 München (DE)
(72) Erfinder: Jährling, Peter, Dipl.-Ing., D-8501 Puschendorf (DE); Schweikert, Eugen, D-8526 Bubenreuth (DE)

(56) Entgegenhaltungen:
- DE-A- 3 605 664

## Beschreibung

Die Erfindung betrifft eine Anordnung zum transkutanen Ein- oder Nachfüllen von flüssigen Medikamenten in einen Medikamentenspeicher eines implantierbaren Medikamentendosiergerätes, mit einer Kanüle zum Durchstechen eines den Medikamentenspeicher nach außen abdichtenden Nachfüllseptums.

Bekannte implantierbare Medikamentendosiergeräte z.B. für Insulin, Cytostatica oder Schmerzmittel, weisen einen Medikamentenspeicher auf, der in bestimmten, vom spezifischen Medikamentenbedarf des Patienten abhängigen zeitlichen Intervallen nachgefüllt werden muß. Üblicherweise erfolgt diese Nachfüllung transkutan mit einer Injektionsspritze, deren Kanüle durch die Haut und ein darunter liegendes, den Medikamentenspeicher nach außen hin abdichtendes Nachfüllseptum hindurchgestochen wird. Eine entsprechende Anordnung ist aus der DE-A-36 05 664 bekannt.

Der Erfindung liegt die Aufgabe zugrunde, eine sichere Nachfüllung zu ermöglichen, wobei insbesondere die Gefahr eines Abbrechens, Abknickens oder Herausrutschens der Kanüle aus der Einstichstelle und damit verbunden die Gefahr von Fehlinjektionen vermindert werden soll.

Gemäß der Erfindung wird die Aufgabe dadurch gelöst, daß bei der Anordnung der eingangs angegebenen Art an dem von der Einstichseite abgewandten Ende der Kanüle ein flexibler Schlauch angeschlossen ist, der von einem Durchstichseptum abgeschlossen ist. Mit der Kanüle wird die Haut und das darunterliegende Nachfüllseptum des implantierten Medikamentendosiergerätes penetriert, wobei mit einer Spritze, die durch das den Schlauch abschließende Durchstichseptum gestochen wird, Medikamentenreste aus dem Medikamentenspeicher abgesaugt werden können und mit einer weiteren Spritze ein frisches Medikament nachgefüllt werden kann. Zum Absaugen und Nachfüllen wird die Haut des Patienten und das Nachfüllseptum des Medikamentendosiergerätes nur ein einziges Mal durchstochen, wobei durch die flexible Schlauchverbindung zwischen der Einstichstelle mit der Kanüle und der Absaug- bzw. Nachfüllspritze die mechanische Belastung der Kanüle verringert wird. Auf diese Weise wird einem Abbiegen, Abbrechen oder Herausrutschen der Kanüle verbunden mit der Gefahr von Verletzungen oder Fehlinjektionen vorgebeugt. Beim Absaugen der Medikamentenreste entsteht in der Anordnung ein Unterdruck, der beim nachfolgenden Einfüllen des frischen Medikamentes eine Saugwirkung hervorruft, die das Nachfüllen erleichtert. Außerdem wird durch den Unterdruck verhindert, daß bei dem Nachfüllvorgang im Falle einer Leckage das Medikament aus der Anordnung austritt und zu einer unter Umständen lebensgefährlichen Fehlinjektion führt. Um den Unterdruck in der Anordnung aufrechterhalten zu können, sind in vorteilhafter Weise jeweils der Schlauch und das Durchstichseptum druckfest gegenüber dem Unterdruck innerhalb der Anordnung ausgebildet.

Entsprechend einer vorteilhaften Weiterbildung der erfindungsgemäßen Anordnung ist vorgesehen, daß im Verlauf des Schlauches zwischem dem Durchstichseptum und der Kanüle eine Filteranordnung liegt und daß im Bereich zwischen der Kanüle und der Filteranordnung ein weiterer flexibler Schlauch mit der Kanüle in Verbindung steht, der von einem weiteren Durchstichseptum abgeschlossen ist. Dabei erfolgt das Absaugen der Medikamentenreste über den weiteren Schlauch, während das Nachfüllen über den Schlauch mit der zwischengeschalteten Filteranordnung vorgenommen wird, so daß verhindert wird, das im einzufüllenden Medikament möglicherweise enthaltene Partikel in das Medikamentendosiergerät gelangen.

Eine wirksame Rückhaltung von in dem nachzufüllenden Medikament enthaltenen Partikeln sowie von in der Filteranordnung selbst durch Faserablösung gebildeten Partikeln wird in vorteilhafterweise dadurch erzielt, daß die Filteranordnung als wenigstens zweilagiges Membranfilter ausgebildet ist, dessen dem Durchstichseptum nähere erste Membran aus einem gegen die Fließrichtung des von dem Durchstichseptum herkommenden Medikaments orientierten Polypropylen-Faservlies und dessen zweite Membran aus einem Tressengewebe besteht. Dabei besitzt vorzugsweise die erste Membran bei einer Flächengröße von etwa 1 cm², einem Medikamentenfluß von 50 - 120 ml pro Minute und einer maximalen Druckdifferenz von 100 mbar eine Rückhalterate von ≧ 5 µm, während die zweite Membran eine Porenweite von 25 µm ohne gerade durchgängige Poren aufweist.

Zur Erläuterung der Erfindung wird im folgenden auf die Figuren der Zeichnung Bezug genommen, von denen
- FIG 1: den prinzipiellen Aufbau eines implantierbaren Medikamentendosiergerätes und
- FIG 2: ein Ausführungsbeispiel der erfindungsgemäßen Anordnung zum Nachfüllen von flüssigen Medikamenten in das Medikmantendosiergerät zeigen.

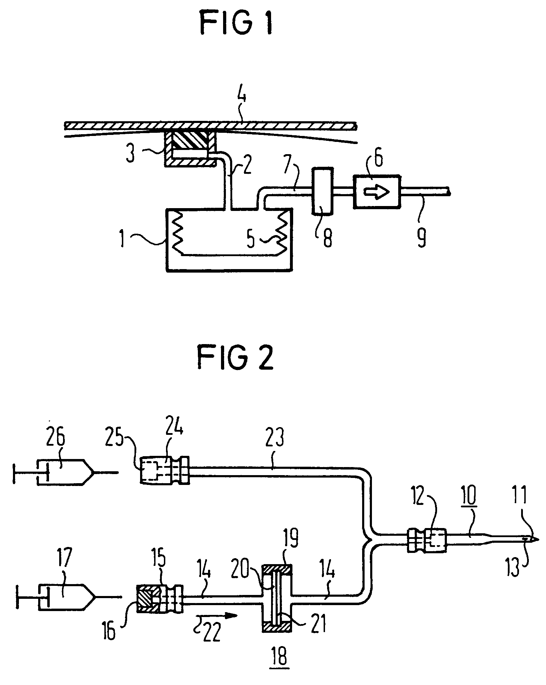

Bei dem in FIG 1 vereinfacht dargestellten prinzipiellen Aufbau eines implantierbaren Medikamentendosiergerätes ist ein Medikamentenspeicher (1) über einen Einfüllschlauch (2) mit einem sog. Nachfüllseptum (3) verbunden, welches unter der Haut (4) eines Lebewesens so angeordnet ist, daß ein flüssiges Medikament, wie z.B. Insulin, mittels einer Kanüle durch das Nachfüllseptum (3) hindurch in ein Membranbalgreservoir (5) des Medikamentenspeichers (1) nach dessen Entleerung einfüllbar ist. Eine Medikamentendosierpumpe (6) saugt das gespeicherte Medikament über einen Verbindungsschlauch (7) ggfs. durch ein Filter (8) hindurch in dosierten Portionen ab und führt diese einem Katheter (9) zu, der die Medikamentenportionen an eine geeignete Stelle im Körper des Lebewesens transportiert und appliziert.

FIG 2 zeigt ebenfalls vereinfacht dargestellt eine Anordnung zum Nachfüllen des flüssigen Medikaments in den Medikamentenspeicher (1) des in FIG 1 gezeigten Medikamentendosiergerätes. Die Anordnung weist eine Kanüle (10) zum Durchstechen des Nachfüllseptums (3) auf, die an ihrem einstichseitigen Ende zu einer kegelförmigen Spitze (11) ausgezogen ist und an ihrem hinteren Ende in einer griffartigen Halterung (12) aus sterilisierbarem Werkstoff, beispielsweise Polycarbonat, befestigt ist. In einem Abstand zur Spitze (11), dem sog. Einstichhub ist die Kanüle (10) mit einer seitlichen Öffnung (13) versehen, deren Außenkanten verrundet sind. Damit wird erreicht, daß das Septummaterial beim Einstechen der Kanüle (10) weder ausgestanzt wird noch Schnittverletzungen erleidet, sondern durch Materialverdrängung kreisrund elastisch aufgeweitet wird.

Die Halterung (12) dient u.a. auch zur Verbindung der Kanüle (10) mit einem flexiblen Schlauch (14), der in einen Handgriff (15) einmündet und dort mit einem Durchstichseptum (16) abgeschlossen ist. Durch dieses Durchstichseptum (16) hindurch läßt sich das nachzufüllende Medikament mittels einer Nachfüllspritze (17) durch die Anordnung hindurch bis in den Medikamentenspeicher (1) hinein applizieren. In den Verlauf des Schlauches (14) ist eine Filteranordnung (18) eingefügt; dabei handelt es sich um ein zweilagiges Membranfilter mit zwei in einem ringförmigen Halterahmen (19) befestigten Membranen (20 und 21), von denen die in Fließrichtung (22) des eingefüllten Medikamentes gesehen erste Membran (20) aus einem gegen die Fließrichtung (22) orientierten Polypropylen-Faservlies mit einem Stützvlies und die stromabwärts angeordnete Membran (21) aus einem Tressengewebe aus Polypropylen besteht. Die Flächengröße der ersten Membran beträgt ca. 1 cm², wobei die Dichte der ersten Membran (20) so gewählt ist, daß diese bei einem Medikamentendurchfluß von 50 - 120 ml pro Minute und einer maximalen Druckdifferenz von 100 mbar eine Rückhalterate von ≧ 5 µm besitzt. Die zweite Membran (21) weist aufgrund ihrer Webart nicht gerade durchgehende Poren mit einer Weite von ca. 25 µm auf, wodurch von der ersten Membran (20) möglicherweise abbrechende Fasern aufgefangen werden. Neben einem guten Partikelrückhaltevermögen wird damit ein nur niedriger Fülldruck (ca. 10 mbar) und eine kurze Füllzeit (ca 1 Min) erreicht.

Im Bereich zwischen der Filteranordnung (18) und der Kanüle (10) ist diese über einen Schlauchabzweig mit einem weiteren flexiblen Schlauch (23) verbunden, der in einen weiteren Handgriff (24) einmündet und dort mit einem weiteren Durchstichseptum (25) abgeschlossen ist. Durch dieses Durchstichseptum (25) hindurch lassen sich vor dem eigentlichen Nachfüllen mittels einer Absaugspritze (26) Medikamentenreste aus dem Medikamentenspeicher (1) durch die gesamte Anordnung hindurch absaugen. Die Schläuche (14 und 23) bestehen aus medikamentenverträglichem und gegenüber einem Unterdruck in der Anordnung druckfesten Material z.B. Polypropylen oder Polyethylen. Die Durchstichsepten (16 und 25) bestehen vorzugsweise aus Bromobutylgummi mit einer unterdruckfesten Dicke von mindestens 1 mm.

Das Entleeren und Wiederbefüllen des Medikamentenspeichers (1) in dem in FIG 1 gezeigten Medikamentendosiergerät geschieht in der Weise, daß zunächst mit der Kanüle (10) die Haut (4) und das darunterliegende Nachfüllseptum (3) des implantierten Medikamentendosiergerätes durchstochen wird und so ein Zugang zu dem Medikamentenspeicher (1) geschaffen wird. Mit Hilfe der Absaugspritze (26), die in das weitere Durchstichseptum (25) eingestochen wird, wird die gesamte Anordnung evakuiert und so das Restvolumen des Medikaments aus dem Medikamentenspeicher (1) entfernt, wobei sich das Membranbalgreservoir (5) zusammenzieht. Die dabei abgesaugte Medikamentenmenge wird verworfen und der Absaugvorgang wiederholt. Anschließend wird die Absaugspritze (26) abgezogen, wobei die Durchstichsepten (16 und 25) gewährleisten, daß der Unterdruck in der Anordnung aufrechterhalten bleibt.

Zum Wiederbefüllen des Medikamentenspeichers (1) wird die luftblasenfrei mit dem frischen Medikament gefüllte Nachfüllspritze (17) in das Durchstichseptum (16) eingestochen, wobei das Medikament durch den in der Anordnung herrschenden Unterdruck eingesaugt wird, bis nach völligem Einsaugen ein Druckausgleich eintritt.

## Patentansprüche

1. Anordnung zum transkutanen Ein- oder Nachfüllen von flüssigen Medikamenten in einen Medikamentenspeicher (1) eines implantierbaren Medikamentendosiergerätes, mit einer Kanüle (10) zum Durchstechen eines den Medikamentenspeicher (1) nach außen abdichtenden Nachfüllseptums (3), **dadurch gekennzeichnet,** daß an dem von der Einstichseite abgewandten Ende der Kanüle (10) ein flexibler Schlauch (14) angeschlossen ist, der von einem Durchstichseptum (16) abgeschlossen ist.

2. Anordnung nach Anspruch 1, **dadurch gekennzeichnet,** daß im Verlauf des Schlauches (14) zwischen dem Durchstichseptum (16) und der Kanüle (10) eine Filteranordnung (18) liegt und daß im Bereich zwischen der Kanüle (10) und der Filteranordnung (18) ein weiterer flexibler Schlauch (23) mit der Kanüle (10) in Verbindung steht, der von einem weiteren Durchstichseptum (25) abgeschlossen ist.

3. Anordnung nach Anspruch 1 oder 2, **dadurch gekennzeichnet,** daß jeweils der Schlauch (14, 23) und das Durchstichseptum (16, 25) druckfest gegenüber einem Unterdruck innerhalb der Anordnung ausgebildet sind.

4. Anordnung nach einem der vorangehenden Ansprüche, **dadurch gekennzeichnet,** daß die Filteranordnung (18) als wenigstens zweilagiges Membranfilter ausgebildet ist, dessen dem Durchstichseptum (16) nähere erste Membran (20) aus einem gegen die Fließrichtung (22) des von dem Durchstichseptum (16) herkommenden Medikaments orientierten Polypropylen-Faservlies und dessen zweite Membran (21) aus einem Tressengewebe besteht.

5. Anordnung nach Anspruch 4, **dadurch gekennzeichnet,** daß die erste Membran (20) bei einer Flächengröße von etwa 1 cm², einem Medikamentenfluß von 50 bis 120 ml pro Minute und einer maximalen Druckdifferenz von 100 mbar eine Rückhalterate von ≧ 5 µm besitzt und die zweite Membran eine Porenweite von 25 µm ohne gerade durchgängige Poren aufweist.

## Claims

1. Arrangement for the transcutaneous filling or refilling of a drug reservoir (1) of an implantable drug-metering device with liquid drugs using a needle (10) for piercing a refilling septum (3) which seals off the drug reservoir (1) towards the outside, characterised in that a flexible tube (14) is connected to the end of the needle (10) facing away from the injection side, which tube is closed off by a piercing septum (16).

2. Arrangement according to Claim 1, characterised in that a filter arrangement (18) is located in the course of the tube (14) between the piercing septum (16) and the needle (10), and in that a further flexible tube (23) is connected to the needle (10) in the region between the needle (10) and the filter arrangement (18), which tube is closed off by a further piercing septum (25).

3. Arrangement according to Claim 1 or 2, characterised in that the tube (14, 23) and the piercing septum (16, 25) are each constructed to be pressure-resistant relative to a negative pressure within the arrangement.

4. Arrangement according to one of the preceding claims, characterised in that the filter arrangement (18) is constructed as an at least two-layered membrane filter whose first membrane (20) which is nearer to the piercing septum (16) consists of a polypropylene fibrous nonwoven material which is oriented counter to the flow direction (22) of the drug coming from the piercing septum (16), and whose second membrane (21) consists of a braided fabric.

5. Arrangement according to Claim 4, characterised in that the first membrane (20), with a surface area of about 1 cm², a drug flow of 50 to 120 ml per minute and a maximum pressure difference of 100 mbar, has a retention rate of ≧ 5 µm, and the second membrane has a pore width of 25 µm without straight-through pores.

## Revendications

1. Dispositif de remplissage ou de réajustement du remplissage transcutané de médicaments liquides, d'un réservoir de médicament (1) d'un appareil implantable d'introduction dosée d'un médicament, comportant une canule (10) destinée à percer un septum de réajustement du remplissage (3) qui établit une étanchéité vis-à-vis de l'extérieur, caractérisé par le fait qu'à l'extrémité de la canule (10), tournée à l'opposé du côté perforable, est raccordé un tuyau flexible (14), qui est fermé par un septum perforable (16).

2. Dispositif suivant la revendication 1, caractérisé par le fait qu'un dispositif formant filtre (18) est disposé entre le septum perforable (16) et la canule (10) dans le tuyau (14) et que dans la zone située entre la canule (10) et le dispositif formant filtre (18), un autre tuyau flexible (23), qui est fermé par un autre septum perforable (25), est raccordé à la canule (10).

3. Dispositif suivant la revendication 1 ou 2, caractérisé par le fait que respectivement le tuyau (14,23) et le septum perforable (16,25) sont agencés de manière à résister à une dépression à l'intérieur du dispositif.

4. Dispositif suivant l'une des revendications précédentes, caractérisé par le fait que le dispositif formant filtre (18) est agencé sous la forme d'un filtre à membrane formé d'au moins deux couches, dont la première membrane (20), qui est la plus proche du septum perforable (16), est constituée par une nappe de fibres de polypropylène, orientée en sens opposé de la direction d'écoulement (22) du médicament qui arrive du septum perforable (16), et dont la seconde membrane (21) est constituée par un tissu tressé.

5. Dispositif suivant la revendication 4, caractérisé par le fait que pour une étendue en surface d'environ 1 cm², un débit du médicament de 50 à 120 ml par minute et une différence maximale de pression de 100 mbars, la première membrane (20) possède un taux de retenue ≧ 5 µm et que la seconde membrane possède des pores d'une taille de 25 µm sans aucun pore traversant rectiligne.
